# FASCICULE DE BREVET EUROPEEN

(11) **EP 1 714 647 B1**
(45) Date de publication et mention de la délivrance du brevet: **15.12.2010**
(21) Numéro de dépôt: 06290636.7
(22) Date de dépôt: 19.04.2006
(51) Int. Cl.: A61K 31/165, A61P 25/00, A61P 25/18, A61P 25/24

(54) **Utilisation de l'agomelatine pour l'obtention de medicaments destines au traitement des troubles bipolaires**
Verwendung des Agomelatins zur Herstellung eines Medikamentes zur Behandlung der bipolaren Erkrankungen
Use of agomelatin in the manufacture of a medicament for the treatment of bipolar disorders

(30) Priorité: 20.04.2005 FR 0503937
(43) Date de publication de la demande: 25.10.2006
(73) Titulaire: Les Laboratoires Servier, 92415 Courbevoie Cedex (FR)
(72) Inventeur: De Bodinat, Christian, 92210 Saint-Cloud (FR); Mocaer, Elisabeth, 92200 Neuilly-Sur-Seine (FR)

(56) Documents cités:
- WO-A-2005/063297
- CHILMAN-BLAIR K ET AL: "AGOMELATINE. ANTIDEPRESSANT TREATMENT OF BIPOLAR DISORDER MELATONIN AGONIST/5-HT2C ANTAGONIST" DRUGS OF THE FUTURE, BARCELONA, ES, vol. 28, no. 1, janvier 2003 (2003-01), pages 7-13, XP009036058 ISSN: 0377-8282
- GRAUL A I: "ANNUAL UPDATE 2003: DRUGS FOR PSYCHIATRIC DISORDERS AND SUBSTANCE ABUSE" DRUGS OF THE FUTURE, BARCELONA, ES, vol. 28, no. 11, 2003, pages 1103-1144, XP008030818 ISSN: 0377-8282

## Description

La présente invention concerne l'utilisation de l'agomélatine ou *N*-[2-(7-méthoxy-1-naphtyl)éthyl]acétamide de formule (I) : 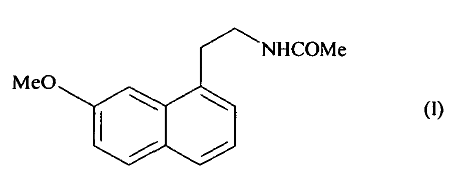 ainsi que ses hydrates, formes cristallines et sels d'addition à un acide ou à une base pharmaceutiquement acceptable, seule ou en association pour l'obtention de médicaments destinés au traitement des troubles bipolaires, notamment des troubles bipolaires de types I et II, et plus particulièrement des troubles bipolaires de type I.

L'agomélatine ou *N*-[2-(7-méthoxy-1-naphtyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

La demanderesse a présentement découvert que l'agomélatine ou *N*-[2-(7-méthoxy-1-naphtyl)éthyl]acétamide, ainsi que ses hydrates, formes cristallines et sels d'addition à un acide ou à une base pharmaceutiquement acceptable, utilisée seule ou en association, possédait des propriétés intéressantes permettant de l'utiliser dans le traitement des troubles bipolaires, notamment des troubles bipolaires de types I et II, et plus particulièrement dans les troubles bipolaires de type I.

Les désordres bipolaires sont un état psychopathologique ayant un impact des plus sévères sur la vie des patients, tant sur le plan social, familial que professionnel. Ils sont caractérisés par la survenue généralement répétée d'épisodes dépressifs, maniaques, hypomanes ou mixtes séparés par des périodes au cours desquelles les sujets sont a priori indemnes de dysfonctionnement psychique majeur. En d'autres termes, les patients souffrant de troubles bipolaires sont marqués par une vulnérabilité à présenter des fluctuations marquées de l'humeur de manière récurrente. Les caractéristiques des accès et leur évolution dans le temps permet de distinguer plusieurs formes cliniques : le trouble bipolaire de type I est le plus typique et se caractérise par un ou plusieurs épisodes maniaques ou mixtes habituellement accompagnés d'épisodes dépressifs majeurs ; le trouble bipolaire de type II quant à lui concerne l'association d'au moins un épisode dépressif majeur et d'un épisode d'hypomanie, forme atténuée de la manie. Le risque de suicide de patients bipolaires est très élevé : 25 à 50 % d'entre eux ont fait au moins une tentative de suicide.

Il n'existe pas actuellement de traitement satisfaisant et reconnu pour les troubles bipolaires. Les traitements de première intention sont généralement les stabilisateurs de l'humeur ou thymorégulateurs, mais ces traitements sont souvent incapables de soulager les symptômes dépressifs (J. Clin. Psychiatry, 2004, 65(4), 569-579). La prescription concomitante d'un antidépresseur, bien que fréquemment utilisée, est une pratique très controversée car ces derniers peuvent déclencher ou aggraver les états maniaques et les états mixtes, et doivent être arrêtés lors de la survenue d'épisode maniaque.
Les antidépresseurs sont en particulier réputés pour favoriser l'induction ou l'accélération des cycles hyperthymiques, pour favoriser deux à trois fois plus la survenue d'un virage maniaque ou hypomaniaque, et enfin leur utilisation prolongée semble mener à une augmentation du nombre d'épisodes dépressifs et maniaques.

La demanderesse a présentement découvert que, de façon surprenante, l'agomélatine pouvait être utilisée dans le traitement des troubles bipolaires, notamment des troubles bipolaires de types I et II, et plus particulièrement dans les troubles bipolaires de type I, seule ou en association.

Les effets attendus de l'utilisation de l'agomélatine dans les troubles bipolaires et plus particulièrement dans les troubles bipolaires de type I étaient, du fait même de son activité dans la dépression, ceux des antidépresseurs décrits dans la littérature comme la paroxétine par exemple.

De façon surprenante, l'agomélatine ne se comporte pas comme un antidépresseur classique, ainsi que cela a pu être observé au cours d'une étude clinique menée auprès de patients souffrants de troubles bipolaires de type I. Ces résultats permettent d'envisager son utilisation, même prolongée, dans les troubles bipolaires, notamment les troubles bipolaires de types I et II, et plus particulièrement dans les troubles bipolaires de type I.

L'invention concerne donc l'utilisation de l'agomélatine, ainsi que ses hydrates, formes cristallines et sels d'addition à un acide ou à une base pharmaceutiquement acceptable, seule ou en association, pour l'obtention de compositions pharmaceutiques destinées au traitement des troubles bipolaires, notamment des troubles bipolaires de type I et II, et plus particulièrement des troubles bipolaires de type I.

L'invention concerne également l'association entre l'agomélatine et un stabilisateur d'humeur ou thymorégulateur pour l'obtention de compositions pharmaceutiques destinées au traitement des troubles bipolaires, notamment des troubles bipolaires de types I et II, et plus particulièrement des troubles bipolaires de type I.
Les stabilisateurs d'humeur ou thymorégulateur selon l'invention concernent le lithium, les antiépileptiques, comme par exemple la carbamazépine, le valproate et la lamotrigine .....
Plus particulièrement, le stabilisateur d'humeur ou thymorégulateur de l'association selon l'invention sera le lithium ou le valproate.
Lorsque l'agomélatine est associée à un stabilisateur d'humeur, elle pourra être administrée avant, pendant ou après le stabilisateur d'humeur, pourvu que l'intervalle de temps entre les deux administrations permette d'obtenir l'effet synergique attendu sur le système nerveux central. Lors d'une administration simultanée des deux composants de l'association selon l'invention, on préférera une composition pharmaceutique contenant l'agomélatine et l'agent stabilisateur d'humeur, en combinaison avec un ou plusieurs excipients pharmaceutiquement acceptables.

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc...

Outre l'agomélatine et le stabilisateur d'humeur qui lui est éventuellement associé, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 1 mg et 50 mg d'agomélatine par 24 heures.

De manière préférentielle, la dose journalière d'agomélatine sera de 25 mg par jour.

### Composition pharmaceutique :

**Formule de préparation pour 1000 comprimés dosés à 25 mg :**

| | |
|---|---|
| N-[2-(7-méthoxy-1-naphtyl)éthyl]acétamide | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Etude clinique :

Cette étude a été réalisée sur 21 patients souffrants de troubles bipolaires de type I, traités depuis au moins 6 mois par du lithium (n = 14), ou du valproate (n = 7). Un traitement de 6 semaines par l'agomélatine (25 mg/jour) a été réalisé. Parmi les patients, 19 ont continué le traitement au-delà des 6 semaines de traitement aigü, et 11 d'entre eux ont poursuivi le traitement jusqu'à 1 an. Les résultats obtenus ont montré que 81% des patients avaient répondu positivement au traitement, avec environ 50% au bout d'une seule semaine de traitement. Seulement trois épisodes maniaques ou hypomaniaques ont été observés après les 6 semaines de traitement, taux tout à fait compatible avec ceux rencontrés avec un stabilisateur d'humeur, démontrant bien que l'action positive de l'agomélatine sur les patients bipolaires de type I n'est pas corrélée à une augmentation des incidences maniaques et/ou hypomaniaques même sur une longue durée de traitement.

## Revendications

1. Utilisation de l'agomélatine ou *N*-[2-(7-méthoxy-1-naphtyl)éthyl]acétamide ainsi que ses hydrates, formes cristallines et sels d'addition à un acide ou à une base pharmaceutiquement acceptable, pour l'obtention d'un médicament destiné au traitement des troubles bipolaires.

2. Utilisation de l'agomélatine ou *N*-[2-(7-méthoxy-1-naphtyl)éthyl]acétamide ainsi que ses hydrates, formes cristallines et sels d'addition à un acide ou à une base pharmaceutiquement acceptable, pour l'obtention d'un médicament destiné au traitement des troubles bipolaires de type I.

3. Compositions pharmaceutiques contenant de l'agomélatine ou un de ses hydrates, formes cristallines et sels d'addition à un acide ou à une base pharmaceutiquement acceptable, seule ou en combinaison avec un ou plusieurs excipients pharmaceutiquement acceptables, destinées au traitement des troubles bipolaires.

4. Compositions pharmaceutiques contenant de l'agomélatine ou un de ses hydrates, formes cristallines et sels d'addition à un acide ou à une base pharmaceutiquement acceptable, seule ou en combinaison avec, un ou plusieurs excipients pharmaceutiquement acceptables, destinées au traitement des troubles bipolaires de type I.

5. Associations contenant de l'agomélatine ou un de ses hydrates, formes cristallines et sels d'addition à un acide ou à une base pharmaceutiquement acceptable, et un agent thymorégulateur.

6. Associations selon la revendication 5 dans laquelle l'agent thymorégulateur est le lithium.

7. Associations selon la revendication 5 dans laquelle l'agent thymorégulateur est le valproate.

8. Compositions pharmaceutiques contenant une association selon l'une des revendications 5 à 7 seule ou en combinaison avec un ou plusieurs excipients pharmaceutiquement acceptables.

9. Compositions pharmaceutiques selon la revendication 8 destinées au traitement des troubles bipolaires.

10. Compositions pharmaceutiques selon la revendication 8 destinées au traitement des troubles bipolaires de type I.

11. Utilisation d'une association selon l'une des revendications 5 à 7 pour l'obtention de compositions pharmaceutiques destinées au traitement des troubles bipolaires.

12. Utilisation d'une association selon l'une des revendications 5 à 7 pour l'obtention de compositions pharmaceutiques destinées au traitement des troubles bipolaires de type I.

## Claims

1. Use of agomelatine, or *N*-[2-(7-methoxy-1-naphthyl)ethyl]acetamide, and also its hydrates, crystalline forms and addition salts with a pharmaceutically acceptable acid or base, in obtaining a medicament intended for the treatment of bipolar disorders.

2. Use of agomelatine, or *N*-[2-(7-methoxy-1-naphthyl)ethyl]acetamide, and also its hydrates, crystalline forms and addition salts with a pharmaceutically acceptable acid or base, in obtaining a medicament intended for the treatment of bipolar disorders of type I.

3. Pharmaceutical compositions comprising agomelatine or one of its hydrates, crystalline forms or addition salts with a pharmaceutically acceptable acid or base, on its own or in combination with one or more pharmaceutically acceptable excipients, intended for the treatment of bipolar disorders.

4. Pharmaceutical compositions comprising agomelatine or one of its hydrates, crystalline forms or addition salts with a pharmaceutically acceptable acid or base, on its own or in combination with one or more pharmaceutically acceptable excipients, intended for the treatment of bipolar disorders of type I.

5. Associations comprising agomelatine or one of its hydrates, crystalline forms or addition salts with a pharmaceutically acceptable acid or base, and a thymoregulatory agent.

6. Associations according to claim 5, wherein the thymoregulatory agent is lithium.

7. Associations according to claim 5, wherein the thymoregulatory agent is valproate.

8. Pharmaceutical compositions comprising an association according to one of claims 5 to 7, on its own or in combination with one or more pharmaceutically acceptable excipients.

9. Pharmaceutical compositions according to claim 8 intended for the treatment of bipolar disorders.

10. Pharmaceutical compositions according to claim 8 intended for the treatment of bipolar disorders of type I.

11. Use of an association according to one of claims 5 to 7 in obtaining pharmaceutical compositions intended for the treatment of bipolar disorders.

12. Use of an association according to one of claims 5 to 7 in obtaining pharmaceutical compositions intended for the treatment of bipolar disorders of type I.

## Patentansprüche

1. Verwendung von Agomelatin oder *N*-[2-(7-Methoxy-1-naphthyl)-ethyl]-acetamid sowie seiner Hydrate, Kristallformen und seiner Additionssalze mit einer pharmazeutisch annehmbaren Säure oder Base zur Herstellung eines Arzneimittels zur Behandlung von bipolaren Störungen.

2. Verwendung von Agomelatin oder *N*-[2-(7-Methoxy-1-naphthyl)-ethyl]-acetamid sowie seiner Hydrate, Kristallformen und seiner Additionssalze mit einer pharmazeutisch annehmbaren Säure oder Base zur Herstellung eines Arzneimittels zur Behandlung von bipolaren Störungen des Typs I.

3. Pharmazeutische Zubereitungen enthaltend Agomelatin oder eines seiner Hydrate, Kristallformen und Additionssalze mit einer pharmazeutisch annehmbaren Säure oder Base, allein oder in Kombination mit einem oder mehreren pharmazeutisch annehmbaren Hilfsstoffen, für die Behandlung von bipolaren Störungen.

4. Pharmazeutische Zubereitungen enthaltend Agomelatin oder eines seiner Hydrate, Kristallformen und Additionssalze mit einer pharmazeutisch annehmbaren Säure oder Base, allein oder in Kombination mit einem oder mehreren pharmazeutisch annehmbaren Hilfsstoffen, für die Behandlung von bipolaren Störungen des Typs I.

5. Kombinationen enthaltend Agomelatin oder eines seiner Hydrate, Kristallformen und Additionssalze mit einer pharmazeutisch annehmbaren Säure oder Base und ein thymoregulierendes Mittel.

6. Kombinationen nach Anspruch 5, worin das thymoregulierende Mittel Lithium ist.

7. Kombinationen nach Anspruch 5, worin das thymoregulierende Mittel Valproat ist.

8. Pharmazeutische Zubereitungen enthaltend eine Kombination nach einem der Ansprüche 5 bis 7, allein oder in Kombination mit einem oder mehreren pharmazeutisch annehmbaren Hilfsstoffen.

9. Pharmazeutische Zubereitungen nach Anspruch 8, bestimmt für die Behandlung von bipolaren Störungen.

10. Pharmazeutische Zubereitungen nach Anspruch 8, bestimmt für die Behandlung von bipolaren Störungen des Typs I.

11. Verwendung einer Kombination nach einem der Ansprüche 5 bis 7 zur Herstellung von pharmazeutischen Zubereitungen, die bestimmt sind zur Behandlung von bipolaren Störungen.

12. Verwendung einer Kombination nach einem der Ansprüche 5 bis 7 zur Herstellung von pharmazeutischen Zubereitungen, die bestimmt sind zur Behandlung von bipolaren Störungen des Typs I.
